(19) Europäisches Patentamt / European Patent Office / Office européen des brevets

(11) **EP 3 232 936 B1**

(12) **EUROPEAN PATENT SPECIFICATION**

(45) Date of publication and mention
of the grant of the patent:
**04.09.2019 Bulletin 2019/36**

(21) Application number: **15870665.5**

(22) Date of filing: **03.12.2015**

(51) Int Cl.:
*A61B 6/02* (2006.01)          *A61B 6/03* (2006.01)
*G06T 11/00* (2006.01)          *A61B 6/00* (2006.01)

(86) International application number:
**PCT/US2015/063675**

(87) International publication number:
**WO 2016/099924 (23.06.2016 Gazette 2016/25)**

(54) **METHOD AND SYSTEM FOR PROCESSING TOMOSYNTHESIS DATA**

VERFAHREN UND SYSTEM ZUR VERARBEITUNG VON TOMOSYNTHESEDATEN

PROCÉDÉ ET SYSTÈME DE TRAITEMENT DE DONNÉES DE TOMOSYNTHÈSE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **17.12.2014 GB 201422477**

(43) Date of publication of application:
**25.10.2017 Bulletin 2017/43**

(73) Proprietor: **General Electric Company
Schenectady, NY 12345 (US)**

(72) Inventor: **BERNARD, Sylvain
78530 Buc (FR)**

(74) Representative: **Fennell, Gareth Charles et al
Kilburn & Strode LLP
Lacon London
84 Theobalds Road
London WC1X 8NL (GB)**

(56) References cited:
EP-A1- 2 712 551          JP-A- 2007 190 182
US-A1- 2006 291 615          US-A1- 2012 121 064
US-A1- 2012 280 684          US-A1- 2014 112 435
US-B2- 7 363 070

• JUN GE ET AL: "Digital tomosynthesis
mammography: intra- and interplane artifact
reduction for high-contrast objects on
reconstructed slices using a priori 3D
geometrical information", SPIE DEFENSE AND
SECURITY SYMPOSIUM; 16-20 MARCH 2008;
ORLANDO, FLORIDA, UNITED STATES, vol.
6512, March 2007 (2007-03), page 65124Q,
XP055377248, US ISSN: 0277-786X, DOI:
10.1117/12.708914 ISBN: 978-1-5106-1723-0

EP 3 232 936 B1

**Description**

Technical Field

The present disclosure relates to the field of tomosynthesis. More particularly, the present disclosure relates to a method and system for processing tomosynthesis imaging data. Moreover the disclosure relates to a computer program product and a computer readable medium. The computer readable medium comprises computer-executable instructions, which, when executed by a computer cause the computer to perform each of the method steps of the disclosure.

Background

X-ray imaging systems have become a valuable tool in medical applications such as for the diagnosis of many diseases. As standard screening for breast cancer mammography 2-dimensional (2D) x-ray images are commonly used that record the images on a photographic film. Using standard mammography high lateral resolution, i.e. in an xy-plane may be achieved. However, no depth resolution, i.e. in a z-direction, may be obtained. Since mammograms take only 2D images across the entire breast tissue image superimposition occurs. Hence, lesions may be masked by the tissue above or underneath, or normal structures may mimic a lesion.

In order to realize depth resolution and to eliminate limitations of standard 2D-mammography as tissue superimposition, digital breast tomosynthesis using digital receptors has been developed. In the most common form of tomosynthesis systems a x-ray tube moves in an arc above a stationary detector. A specific problem to mammography is related to the requirement to be able to clinically analyze interesting microcalcifications having a size ranging from 100 $\mu$m to 500 $\mu$m. Therefore, high resolution detectors (typical 100 $\mu$m) are used to acquire images of a high in-plane (x,y)-resolution together with a z-axis resolution sufficient to visualize microcalcifications. The x,y- and z-resolutions depend on parameters as number of projections or angular range covered by the x-ray source. For example as the angular range increases microcalcifications will appear less sharp due to the degradation of the in-plane resolution.

In digital breast tomosynthesis the volume information of an object of interest is usually derived from a series of images, known as projections, which are taken at various angles by one or more x-ray sources. Objects of different heights in a breast display differently in the different projections. The final step in a common tomosynthesis procedure is to reconstruct the projection data to generate a set of cross-sectional images or thin slices that enhance objects from a given height. That is to say the tomosynthesis procedure focuses on getting only a single plane of image points sharp.

Said cross-sectional planes, hereinafter also being referred to as "tomo planes" or tomosynthesis planes are usually provided as thin slices parallel to a detector. The increment between each tomo plane is specified so that the impulse response of the smallest radiologic sign caused by microcalcifications is crossed at least by one plane. The desired separation between tomo planes is typically around 1mm. Given for example a x-ray tube angular motion of 25°, the z-resolution can be set up in the range of 0,5 mm to 1 mm.

Given this relatively high z-resolutions a health professional needs to review a great number of tomosynthesis planes, typically 50 to 100 planes. If for example the tomosynthesis planes are reconstructed with slice separation of 1mm, a 5 cm compressed breast tomosynthesis will have 50 reconstructed tomo planes. This is in contrast to reviewing a single 2D-standard mammogram. A further factor that can impair the usefulness of the described digital tomosynthesis imaging procedure is the relative difficulty to store all information.

US 8 285 020 proposes to integrate standard 2D mammography images and digital breast tomosynthesis images of a same object of interest. The corresponding display system allows simultaneously acquiring and reviewing standard mammograms and a set of tomo images. With such an arrangement, a medical professional or radiologist may utilize existing expertise gained from reviewing mammograms to more efficiently view the tomosynthesis data set. However, since the average dose from tomosynthesis imaging is approximately the same as mammogram 2D imaging the radiation exposure is roughly doubled. Further, there is the problem that the high resolution of numerous reconstructed planes require substantial reviewing time by the health professional.

US 2012/0121064 discloses a method for rendering 2D image slabs (slices of large thickness) from 3D tomosynthesis images.

Further, a problem with common tomosynthesis data processing methods is that long processing times are required and the network bandwidth and archiving systems may be overloaded. Thus, there is the need to reduce processing time and optimize data flow.

Another problem of conventionally processed tomosynthesis images is tomographic blur and artifacts. Accordingly, there is the need to improve image quality and noise management, to offer tomosynthesis images for improved diagnostic performance and facilitate lesion identification by a health professional.

Summary of the invention

The present invention is defined by the appended claims.

At least one of the above embodiments provides one or more solutions to the problems and disadvantages of the background art. One advantage that may be realized in the practice of some embodiments of the described methods and systems is that workflow and dataflow efficiencies in processing tomosynthesis imaging data are improved. Another advantage is that the data overload due to the planes'z- resolution can be reduced, while increasing the (x,y) resolution. Other technical advantages of the present disclosure will be readily apparent to one skilled in the art from the following description and claims. Various embodiments of the present application obtain only a subset of the advantages set forth. No single advantage is critical to the embodiments. Any claimed embodiment may be technically combined with any other claimed embodiments.

Brief description of the Drawings

The accompanying drawings illustrate exemplary embodiments of the disclosure and serve to explain, by way of example, the principles of the disclosure.

FIG. 1 is a diagrammatic illustration of a work and data flow for reviewing tomosynthesis images;

FIG. 2 is a diagrammatic illustration of a method for obtaining images of an object of interest according to an exemplary embodiment of the disclosure compared to an alternative non-preferred method;

FIG. 3 shows display images according to an exemplary embodiment of the disclosure compared to a display image without processing according to an exemplary embodiment of the disclosure;

FIG. 4 shows flow charts of two methods for obtaining images of an object of interest according to exemplary embodiments of the disclosure;

FIG. 5 shows a diagrammatic illustration of a method for obtaining images according to another exemplary embodiment of the disclosure;

FIG. 6 shows a flow chart of a method for obtaining images according to another exemplary embodiment of the disclosure;

FIG. 7 shows method steps for obtaining images of an object according to another exemplary embodiment of the disclosure;

FIG. 8 is a diagrammatic illustration of a large object in consecutive volume portions according to an exemplary embodiment of the disclosure;

FIG. 9 is a diagrammatic illustration of a method for obtaining images of an object of interest according to another exemplary embodiment of the disclosure;

FIG. 10 shows images obtained with and without processing according to a method of an exemplary embodiment of the disclosure; and

FIG. 11 shows a diagrammatic illustration of a system for obtaining images of an object of interest according to an exemplary embodiment of the disclosure.

Detailed description

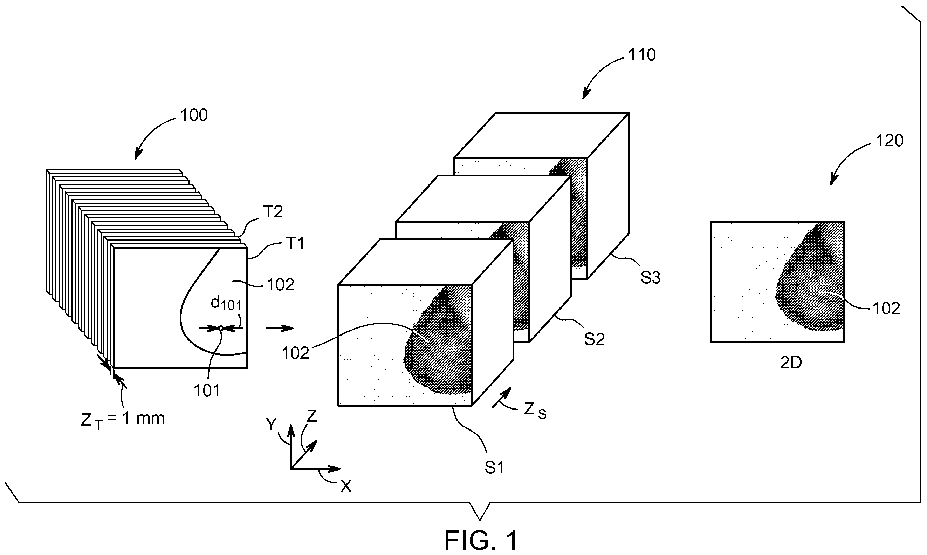

FIG. 1 is a diagrammatic illustration of a work and data flow for reviewing images of an object of interest. The object of interest shown in FIG. 1 is a female breast 102. FIG. 1 shows schematically a stack 100 of a plurality of tomo planes T1 and T2 as obtained and to be reviewed by a health professional.

The thin tomo planes T1 and T2 are characterized by a x,y- and z resolution. When reconstructing a tomo plane T1 from a plurality of 2D projection images, the spatial x,y-resolution of T1 is set to the projections' resolution (100 $\mu$m). Moreover, each tomo plane T1, T2 has a z-resolution $z_T$ of about 1 mm. This z-resolution of 1mm results in a large amount of data - up to 100 planes T1, T2 - to be reviewed and archived.

The z-resolution $z_T$ is set up to 1mm, to cross a microcalcification 101 having diameters $d_{101}$ typically in the order of 100 $\mu$m to 500 $\mu$m. In this way health professionals or radiologists can make in each of the numerous images a fine analysis of microcalcifications. However, this is a time-consuming and tedious process and due to the high spatial x,y,z-resolution a lot of processing time and archiving capacities is needed.

To alleviate the reviewing by health professionals, the system of FIG. 1 provides, in addition to the display of tomo planes T1, T2, an image of a standard mammogram 120to which health professionals are already used to. It is well understood that looking through the stack of tomo planes 100 slice-by-slice takes more time than viewing a single 2D mammogram. Hence, radiologists may use the well known mammogram 120 besides looking for interesting objects in selected tomo planes T1, T2.

In addition to the 2D mammogram 120, an image set 110 of so-called slabs S1, S2 and S3 can be reviewed on demand by the user. The slab S1 is generated by combining two tomo planes T1 and T2. The z-resolution $z_s$ of slab S1 is 2mm instead of 1mm. However, slabbing techniques for thicker slabs according to a non-preferred embodiment of the disclosure suffer from a reduction in visibility of the investigated structures. Due to the problem of noise and artifacts it is still necessary to provide sharp tomo planes T1 and T2 in addition. However, this double image presentation needs both processing time and storage capacities.

Due to the limited angular range, tomosynthesis suffers in general from substantial out-of plane artifacts, which limit the clear definition of the size and depth of structures out of the focus plane. By using thicker image volumes review time can be reduced, but there is the problem that the image quality can be adversely affected.

Since it is difficult to rapidly detect clinically relevant structures in fifty (50) let alone one hundred (100) tomo planes, known tomosynthesis methods represent to a radiologist an increased workload compared to conventional 2D mammogram reviews. From these disadvantages the need derives to provide less images to be reviewed by health professionals and at the same time to maintain high quality images showing clinically relevant structures sufficiently sharp.

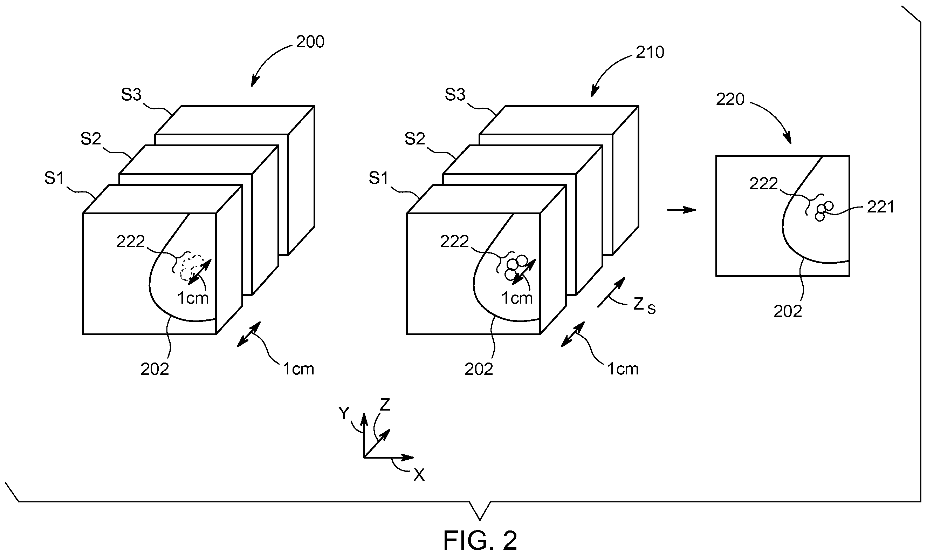

In FIG. 2, a plurality of slabs 210 according to an exemplary embodiment of the disclosure is diagrammatically illustrated in comparison to a plurality of alternative non-preferred slabs 200. Both processing methods generate slabs S1, S2 and S3 having an extension of 1 cm in the z-direction. However, the images of slabs 200 and the visualization of a cluster 222 of microcalcifications suffer from high noise levels and artifacts. With such a relatively low image quality associated with slabs 200, sharper images with reduced blurr are still needed.

A reduction of artifacts is realized according to a method of the disclosure, wherein the 1 cm thick 3D volume portion S1 of slabs 210 show the same cluster 222 of microcalcifications with sharp edges. Further, this slab rendering method shown in slabs 210 makes structures visible as if each of them were in their focus plane. In other words, the impulse response of a point object as a microcalcification is independent of the vertical position in the 3D volume portion. If a point object is imaged by a tomosynthesis system, then the impulse response of the point object can be described by the so-called point spread function (PSF). Generally the degree of blurring (spreading) of a point object is a measure for the quality of an imaging system. According to an embodiment of the disclosure shown in slabs 210, each point object has less blurr than in slabs 200 regardless of the vertical position of the point object in direction $z_S$ of slab S1.

Moreover, the embodiments of slabs 210 and 220 according to the disclosure are characterized by a reduced z-resolution. The 3D volumes 210 or 220 are obtained such that the dataset z-resolution of the slab $z_S$ and/or the 3D volume portion thickness is set to the average size of a lesion being 1 cm. This has the advantage that health professionals may reliably detect clinically relevant structures as lesions, which have an average extension of 1 cm (see double arrow in Fig. 2). Since a great number of cancers detected by mammography correspond to clustered microcalcifications and masses, a health professional is usually not looking for individual microcalcifications but rather for clusters 222 of several calcifications as well as masses having an average size of 1cm. That is to say it is sufficient for diagnosis of early breast cancer to set $z_S$ to the size of typical lesions.

Since lesions are larger than individual calcifications, the tomo data set can significantly be reduced. With a reduced z-resolution 3D volumes S1, S2 as shown in the set 210 require less storage. The example shown in FIG. 2 shows 1 cm thick slabs S1 and S2. In the case of 1 cm thick and 0,5 mm spaced planes, the corresponding slabs require about 10 times less data.

Another advantage of 3D volume portions according to embodiments 210 and 220 of the disclosure is the high image quality. The sharpness of the point in the slabbed three dimensional (3D) volume S1 of set 210 allows reliable detection of potential lesions by radiologists. By generating and displaying 10 slabs instead of 100 standard tomo the review time of radiologists can be significantly accelerated.

FIG. 2 shows as an additional 2D like image 220 that may be displayed on demand on the review workstation by a health professional. This image is representative for a 3D volume, which is a slab of the entire volume of the object of interest being a female breast 202. The impulse response of a point object is not dependent of the vertical position in this full volume overview. Hence, this 2D like image 220 shows the entire volume of the object 202 similar to a standard mammogram to which the health professional is used to.

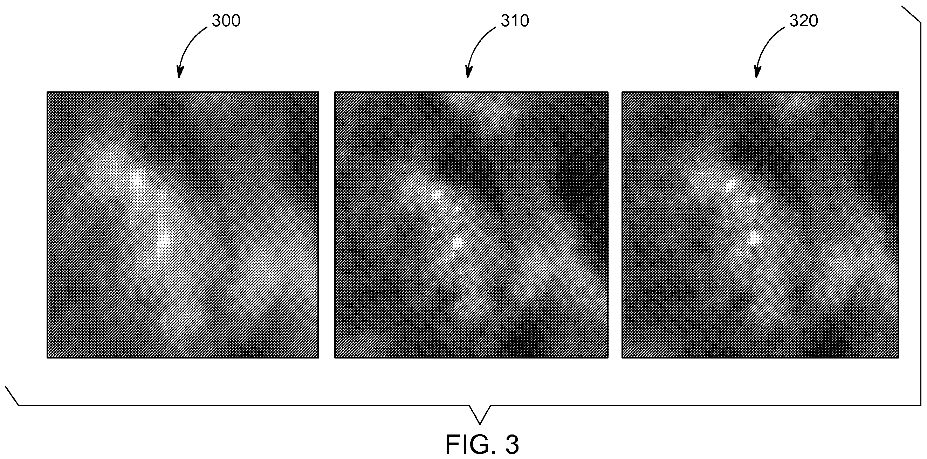

FIG. 3 as in FIG. 2, depicts exemplary images obtained by a non-preferred slabbing method compared to images obtained by a method according to an exemplary embodiment of the disclosure. The 3D volume image 300 shows a slab obtained by using maximum intensity projection or maximum intensity pixel (MIP) technique. The slabbing method of image 300 produces artifacts and blurred structures, such that the structures cannot be seen clearly.

In contrast to the obtained image 300, the images 310 and 320 are images obtained according to methods of the disclosure, wherein the same structures of interest are imaged. Hence the quality of images 310 and 320 is enhanced, compared to image 300, since artifacts and noise are significantly reduced. The image 310 shows an example of a generated slabbed 3D volume portion, whereas image 320 shows a plane of the same object of interest. This 2D image 320 has a comparable high resolution to a standard mammogram, which the health professional is used to analyzing.

A further advantage of the methods according to the disclosure is that the x,y-resolution of the plane can be increased leading to better fine structures depiction. Displaying more precisely shapes of microcalcifications indeed provides clinical information to the radiologist. In common slabs the x,y-resolution is set to the resolution of the acquired 2D projections. The 2D projections are back-projected, wherein at a given height the back-projected samples are independent from one projection to the other. Therefore the x,y-resolution versus the projections' resolution can be increased. In this way the x,y-resolution can be computed such that the plane x,y-resolution is higher than the sampling spacing of the detector. For example, if the detector has a resolution of 100 micron a x,y-resolution of 50 or 70 micron can be obtained. Due to a higher x,y-resolution, microcalcifications represented by bright image points in the obtained images 310 or 320 can be recognized more clearly in contrast to images generated according to a non-preferred method as shown, for example, in image 300.

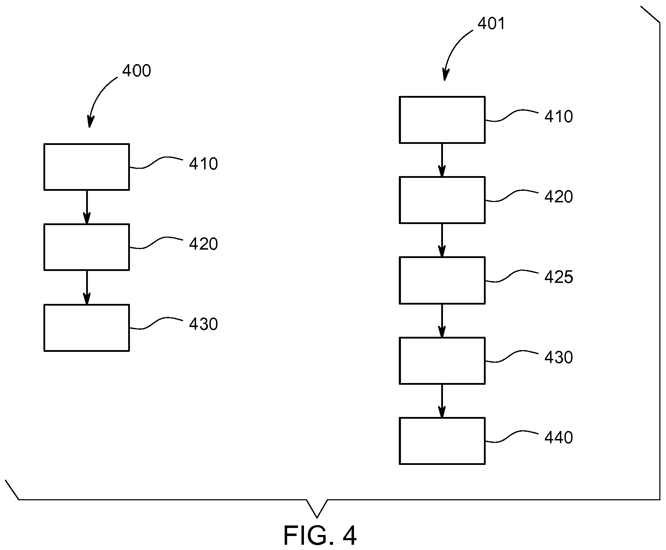

FIG. 4 shows flow charts for processing methods to obtain images according to alternative embodiments of the disclosure. The method 400 comprises a first method step 410 to acquire a plurality of 2D projection images of the object of interest in a plurality of orientations. In a second method step 420 at least one 3D volume of the object of interest from the plurality of 2D projections images is generated, wherein the response of a point object is independent of the object's position in the vertical direction (z-axis) of the 3D volume portion. In a third method step 430 at least one 3D volume portion is displayed. The obtained images can be reviewed by the health professional in a workstation configured to review one or more 3D volume portions for diagnosis.

While the exemplary embodiment 400 shows three method steps, additional steps can be taken, wherein the steps can be taken in any order as long as they make technical sense. As used hereinafter, an element or step recited in the singular should be understood as not excluding a plurality of said elements or steps, unless such a step is explicitly excluded.

For example there are two alternatives in order to generate 3D volume portions. A first method comprises using a reconstruction method that directly generates the at least one 3D volume from the plurality of 2D projection images. According to this embodiment no standard tomosynthesis planes are generated and only 3D volume portion images being thicker than the standard planes may be displayed or optionally a 2D like image. Hence, the data set is significantly reduced compared to data flows further generating and displaying numerous tomo planes in 1mm spacing.

Secondly, there is the possibility to generate 3D volume portions only after reconstructing a plurality of intermediate tomosynthesis planes from the plurality of 2D projection images. According to this embodiment, a plurality of intermediate tomosynthesis planes is preferably generated temporarily for further post-processing. The post-processing algorithm is configured to provide an improved slabbing method for combining at least several intermediate tomosynthesis planes. At least one technical effect of this slabbing method is reducing noise and artifacts compared to image processing methods according to a non-preferred embodiment.

Yet another embodiment of the method according to the disclosure is shown in flow chart 410, wherein the generating step 420 is aimed to generate a set of successive or consecutive 3D volume portions. In the method step 425, said volume portions are generated such that there is an overlap between two consecutive 3D volume portions. In step 430 the at least one of the plurality of overlapping 3D volume portions are displayed to a health professional for review. The workstation for review is configured to show the volume portions consecutively either as a cine loop or any other standard display mode. Due to the high x,y-resolution of the volume portions the image quality is sufficiently high to exclusively base the diagnosis on the set of 3D volume portion images. Hence, the calculation and provision of 1mm spaced tomo-planes is no longer necessary and can be dispensed with. As a further method step 440, the displayed 3D volume portions can be sent to an archive. The method step of archiving (440) images of the at least one 3D volume portion can be performed without archiving the plurality of intermediate planes. Archiving 440 of all slabbed volume portions can be automatically done by a processing unit. Alternatively the health professional may select the volume portions to be archived. Yet another option is to first down sample the high x,y resolution of the images and thereafter to send a reduced data set to the archive.

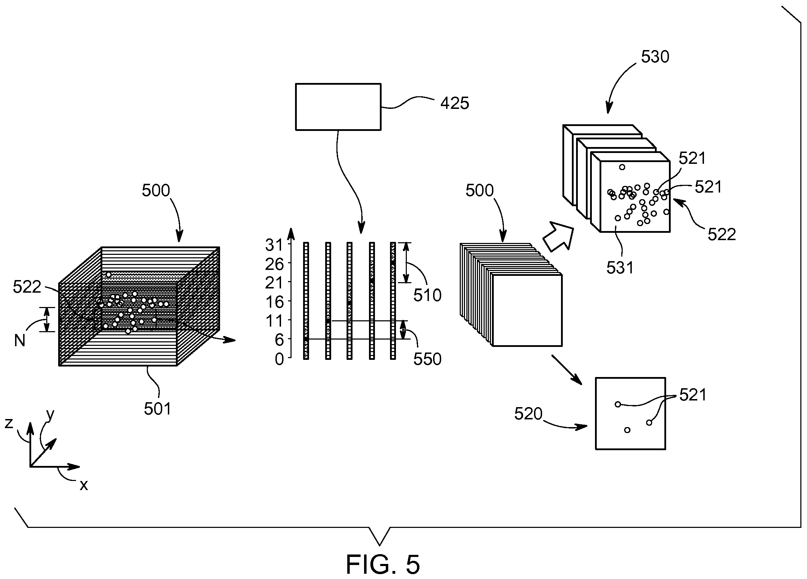

FIG. 5 shows a diagrammatic illustration of a method according to an exemplary embodiment of the disclosure, which is based on the intermediate method step 500 to generate intermediate tomosynthesis planes in form of a so called stack. The method begins with acquiring projection data by scanning an object. Thereafter the method step 500 follows, wherein a plurality of temporary tomosynthesis planes 501 are generated from the plurality of 2D projection images. The intermediate tomosynthesis planes 501 lie parallel to an x,y-plane of a Cartesian coordinate system and are typically located parallel to an array of an detector. The tomosynthesis planes 501 are typically spaced at 1 mm.

Then at least some of the temporary tomosynthesis planes are used to generate at least one 3D- volume portion. One exemplary 3D volume portion is shown having consecutive neighboring thin volume portions having a thickness N (see double arrow). This 3D volume extends in z-direction preferably to the average size of a lesion of about 1 cm. Hence at least 10 intermediate tomosynthesis planes constitute a 3D volume portion or so called overview slab.

When reviewing only a single thin tomosynthesis plane 520, which is retrieved from the stack 500 of tomosynthesis planes, only individual calcifications 521 appear. This reviewing technique impairs the appreciation of a cluster 522, which is not aligned in a single plane. Further, the appearance of masses lying in more than one thin tomosynthesis plane, may also not be recognized by a health professional. By using the overview slabs 510 according to an embodiment of the invention, cluster information is maintained, even if the cluster 522 extends over several tomosynthesis planes 501. Another advantage of the generated 3D volume portions or overview slabs of typically 1 cm thickness is, that the number of images to be reviewed can be reduced.

In order to further improve cluster characterization, 3D volume portions according to an embodiment of the disclosure are combined with neighboring volume portions. In a preferred configuration the thickness of a slabbed 3D volume portion (slab thickness z) is set to 10 mm and the overlap is about 0.5cm. This results in an overlap 550 of about half of the volume portion. The generation of five overlapping volume portions is schematically shown in the illustration of method step 425, wherein each volume portion 510 consists of a central tomosynthesis plane and five tomosynthesis planes on either side, which compose the overview slab 510 composed of originally 11 tomo planes.

The generated thick 3D- volume portions are displayed in method step 530 to a health professional. The reviewer may screen a reduced image set of typically 10 slabbed volume portions 531 compared to a standard set of about 100 tomosynthesis planes 501.

Due to the z-resolution of the 3D volume portions of 1 cm and the overlap of each consecutive slab, the information of cluster and masses are advantageously represented. The method step 530 shows displaying a set of slabs 531, wherein said slab 531 shows a cluster 522 of microcalcifications 521. The overlap of slabs according to method step 425 avoids splitting lesions into consecutive slabs. Even if a cluster is obviously cut at the upper or lower edge of a displayed volume portion 531, the radiologist may toggle to the corresponding adjacent volume portion. Although the imaged calcifications 521 - shown schematically in the slabbed 3D volume portion 531 as white spheres - extend over the entire 3D volume portion 531, a radiologist can clearly identify the cluster 222 in its entirety.

Another potential work flow for a medical professional would be to first scroll through the volume portions 531 and overview slabs to identify potential lesions. Thereafter selected tomosynthesis planes 501 could be displayed in method step 520 for further characterization. Depending on the desired information by a health professional, the 3D volume portions and optionally selected tomosynthesis planes 501 may be archived.

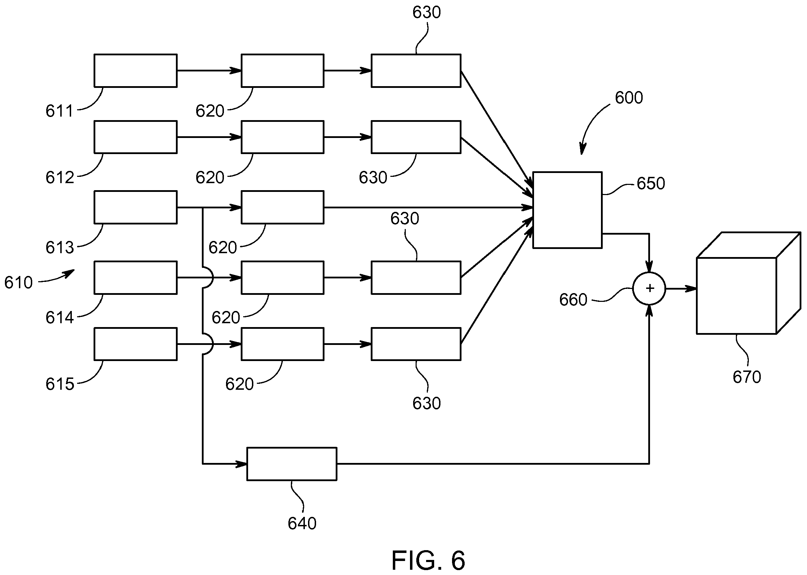

FIG. 6 shows schematically a flow chart for a method with noise and artifacts reduction, wherein as first step 610 a plurality of tomosynthesis planes are generated from the source data (2D projection images). The final overview slab or volume portion for display 670 is composed of six tomosynthesis planes: 611, 612, 613, 614, and 615. The number of tomosynthesis planes is exemplary and may vary. However, an odd number of tomosynthesis planes 611-615 is preferred to allow a central plane 613 to be surrounded by an equal number of tomosynthesis planes on either side (in FIG. 6 611 and 612 on the upper side and 614 and 615 on the lower side).

The central plane 613 and remaining planes 611, 612, 614, 615 are high pass filtered in step 620. According to one embodiment of the disclosure, the cut off frequency of the high pass filter depends on the desired x,y-resolution of the 3D volume. Alternatively, the cut-off frequency of the high pass filter depends on the 3D volume portion z-resolution and/or thickness. In yet another aspect of the disclosure the cut-off frequency of the high pass filter depends on the acquisition system tomosynthesis aperture angle. The generation of a slabbed 3D volume portion further comprises step 630, wherein all tomosynthesis planes surrounding the central plane i.e. planes 611, 612, 614, 615, which compose the final 3D volume, are projected on the central plane. In the next method step 650 the projected planes and the filtered central plane are combined with a maximum intensity projection (MIP) method.

The low frequencies of the overview slab are provided by a further method step 640, wherein the central plane is low pass filtered. The final overview slab is generated in step 660 by combining the MIP processed data (650) to the low pass filtered central plane from method step 640. According to an embodiment of the disclosure, after step 660 an overview slab image is displayed in step 670, which may be archived afterwards.

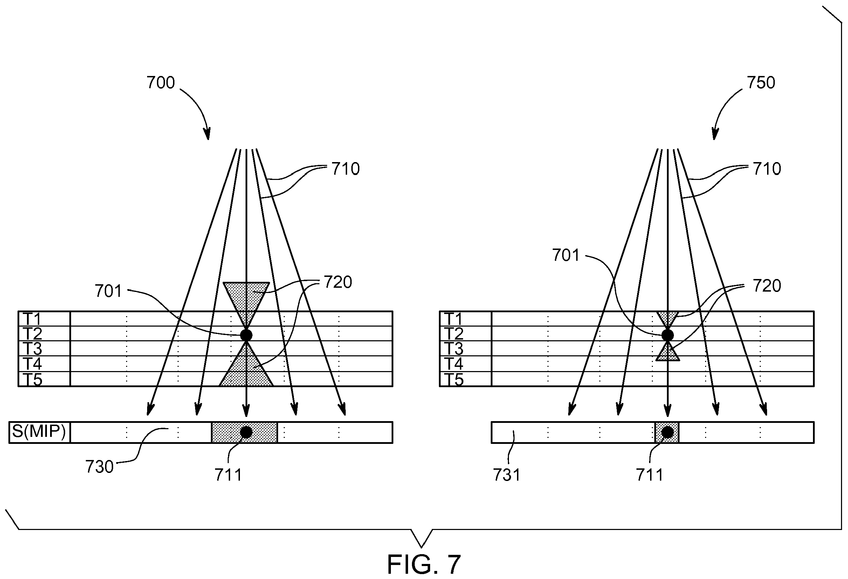

FIG. 7 is a diagrammatic illustration of a slabbed 3D volume S(MIP) using maximum intensity pixel (MIP) technique. The diagram 700 shows a conic MIP technique without filtering. In addition diagram 750 illustrates the conic MIP of diagram 700 with the further method step of high pass filtering.

As first method step a plurality of 2D projection images of the object of interest 701 are acquired and therefrom a plurality tomosynthesis planes T1- T5 are reconstructed. Further, the maximum intensity projection (MIP) method along each projection ray 710 is applied. Along the ray path, which is illustrated by the arrow 710 crossing the punctual object 701, a data point of maximum intensity value is retrieved. Accordingly, in the generated slab S(MIP) only the highest values are represented. However the visualization of point object 701 in the generated slab S(MIP) is affected by noise due to the back projection process. In particular the back projection generates artificial low frequencies, which is illustrated in FIG. 7 by fans 720.

Diagram 750 shows a method for reducing the artifacts formed like fans 720, wherein each plane T1, T2, T3, T4 and T5 is high pass filtered. The high pass filtering step reduces the fan 720 such that each fan 720 is smaller in diagram 750. Accordingly, the generated slabbed 3D volume 731 shows a spherical object of interest 711 with a reduced blurred region compared to 730.

According to an embodiment of the disclosure, each tomosynthesis plane $p$ may be high pass filtered along the x-axis leading to the high pass filtered HP$_p$ image as follows:

$$HP_p(i,j) = p(i,j) - (p * g_\sigma)(i,j) = p(i,j) - \sum_{l=-WinLen}^{WinLen} p(i,j+l) \cdot g_\sigma(l)$$

where $g_\sigma(l) = \frac{1}{\sqrt{2\pi} \cdot \sigma} e^{-\frac{l^2}{2\sigma^2}}$ is a Gaussian filter;

$\sigma$ is the spread of the Gaussian filter and

*WinLen* the window size used for computing the convolution.

The object of the high pass filtering step is to attenuate blur as fans 720 generated by the back-projection process along the x-axis. As shown in FIG. 7 each fan 720 generates blur in the slabbed 3D volume images, when computed with a standard maximum intensity projection method. In a particular embodiment, given $\sigma$ fixed to 4, the blur is attenuated (see smaller fan area in FIG. 7) but large objects bigger than 1mm are removed.

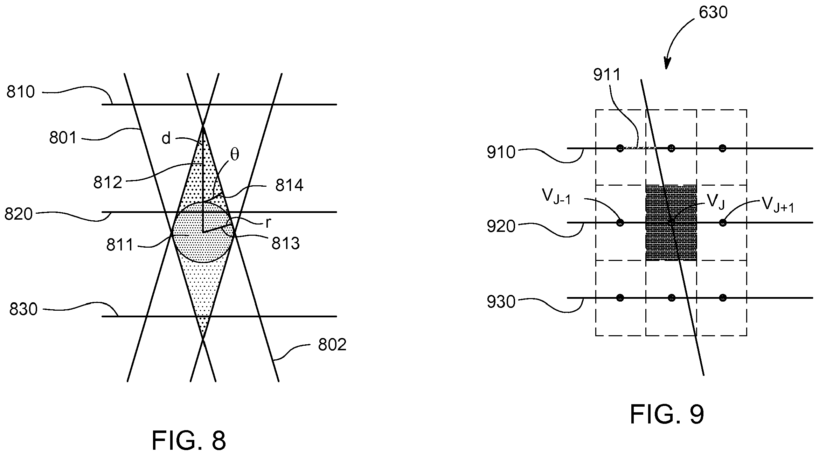

FIG. 8 illustrates a diamond shape representing the main response of a sphere 211 in relation to consecutive central planes. The spherical object 811 with a radius 813 is so large that the object backpropagates widely. FIG. 8 shows three consecutive central planes 810, 820 and 830, wherein the second central plane 820 and the third central plane 830 are crossed by the diamond shaped response of the object 811. Depending on the largeness of the object and the aperture angle θ, either no plane or at least one plane may be crossed by the diamond shaped response of the respective object.

If the aperture angle θ is set to 11.75° and the distance between adjacent central planes is fixed to 5 mm, at least one central plane is crossed by the diamond shaped response of an object, when the diameter of the object 811 is superior to 1mm. If the distance between adjacent planes is larger e.g. 10 mm instead of 5 mm, the radius r needs to be larger such, that the long diagonal of a diamond (2·d) crosses at least one central plane. If a parallel geometry is assumed, wherein source and detector distance is infinite and the rays 801, 802 are parallel, a minimum radius 813 of the sphere 811 can be calculated such that at least one central plane 820 is crossed by the top of the diamond. For radius r and d being half the diagonal of the following radius r can be calculated:

$$\text{r} = \text{d} \cdot \sin(\theta) = 5 \text{ mm} \cdot \sin(11.75) \cong 1 \text{mm}$$

Taken a distance of 5 mm between adjacent central planes; objects having bigger diameters than 1mm can be recovered from the central plane by performing the aforementioned method step of low pass filtering 640. The low frequencies of the slab can be obtained by low-pass filtering the central plane c along the x-axis as follows:

$$Slab_{Low\ Frequencies}(i,j) = (c * g_\sigma)(i,j) = \sum_{l=-WinLen}^{WinLen} c(i,j+l) \cdot g_\sigma(l)$$

Before any further processing of the slab called $Slab_{Low\ Frequencies}(i,j)$ is possible, the high pass filtered planes need to be combined in a so called high frequency slab called $Slab_{High\ Frequencies}$, which is obtainable by plane projections on the central via step 650 as described in more detail below.

The method step 630 is illustrated in FIG. 9, which schematically shows three intermediate tomosynthesis planes 910, 920 and 930 and a response of a point object 911. The central plane 920 comprises voxels $v_j$ and neighboring voxels $v_{j-1}$ and $v_{j+1}$. To carry out a central projection, the voxels through which a particular ray passes during data collection are considered. More particularly, the projection of plane 910 is accomplished with the following method:

The slab pixel ($i^{slab}, j^{slab}$) are projected on the slice plane at the coordinates:

$$x^{plane} = x^{source} + i^{slab} \cdot d$$

$$y^{plane} = y^{source} + j^{slab} \cdot d$$

where $d = \frac{z^{source} - z^{plane}}{z^{source} - z^{slab}}$ and ($x^{source}$, $y^{source}$, $z^{source}$) are the coordinates of a virtual source situated along the x-ray tube trajectory and perpendicular to the detector plane. Conventionally, the x and y directions are within the plane of each plane, whereas the z-direction is perpendicular to the x,y-plane.

The closest slice voxel upper and to the left with respect to ($x^{plane}$, $y^{plane}$) is ($i^{plane}$, $j^{plane}$). The distances between ($x^{plane}$, $y^{plane}$) and ($i^{plane}$, $j^{plane}$) along x-axis and y-axis, respectively are:

$$\Delta x^{plane} = x^{plane} - i^{plane}$$

$$\Delta y^{plane} = y^{plane} - j^{plane}$$

The re-projection value at ($i^{slab}$, $j^{slab}$) is computed by bilinear interpolation using the four nearest neighbour plane pixels of the location ($x^{slice}$, $y^{slice}$) according to the following equation:

$$
\begin{aligned}
HP_p'\left(i^{slab}, j^{slab}\right) &= (1 - \Delta x^{plane}) \cdot (1 - \Delta y^{plane}) \cdot HP_p\left(i^{plane}, j^{plane}\right) \\
&+ \Delta x^{plane} \cdot (1 - \Delta y^{plane}) \cdot HP_p\left(i^{plane} + 1, j^{plane}\right) \\
&+ (1 - \Delta x^{plane}) \cdot \Delta y^{plane} \cdot HP_p\left(i^{plane}, j^{plane} + 1\right) \\
&+ \Delta x^{plane} \cdot \Delta y^{plane} \cdot HP_p\left(i^{plane} + 1, j^{plane} + 1\right)
\end{aligned}
$$

After the high pass filtered planes have been projected $HP_p'$ - planes composing the slab are obtained. As next method step a Maximum Intensity Projection (MIP) method is performed.

An overview slab representing a 3D volume portion according to an embodiment of the disclosure is obtained by a final slab recombination. This slab combination is performed in method step 660 an can be accomplished as follows:

$$Slab(i,j) = Slab_{Low\ Frequencies}(i,j) + Slab_{High\ Frequencies}(i,j)$$

In this way the low pass filtered central plane i.e. $Slab_{Low\ Frequencies}(i,j)$ and all high pass filtered tomosynthesis and projected planes composing the final 3D volume portion i.e. $Slab_{Low\ Frequencies}(i,j)$ are added. Objects with diameters larger than 1mm are recovered by the low-pass filtered central plane.

In summary the above method for reducing review time for radiologists as well as image artifacts comprises at least the following steps. The method begins with step 610 by obtaining a plurality of tomosynthesis planes from 2D projection images. First, a high pass filtering 620 is applied to each plane. Second a central projection of the planes is performed in step 630, and third a maximum intensity projection (MIP) 650 is applied to generate a thicker slab. The central plane is further low pass filtered in method step 640, in order to generate a slab with responses of larger objects. Finally, the slab with high frequencies and the slab with low frequencies are recombined 660 to generate the final overview slab 660.

In this way a plurality of overview slabs may be generated, which have reduced blur and sufficient image quality to identify clinically relevant structures. Due to the improved image quality there is no need to display or archive the data of the intermediate tomosynthesis planes composing each slab.

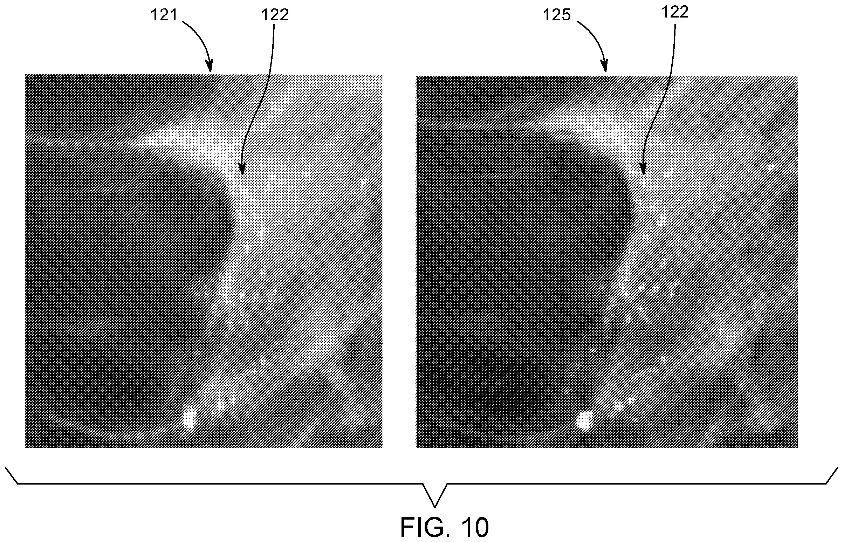

FIG. 10 shows an image according to the processing method of an embodiment of the disclosure as well as an MIP processed image according to an alternative non-preferred method. The image 121 generated according to said non-preferred method shows a lot of blur. When using the disclosed exemplary method for generating the slab, the artifacts could significantly be reduced as shown in image 125. The arrow 122 in image 121 points to calcifications in a blurred environment, wherein the brighter spots representing calcifications are rarely visible. The same calcifications indicated with arrow 122 in the image 125 are sharper in contrast to the image processed by the non-preferred MIP method, wherein blur artifacts are present due to the backprojection process. The image generation according to exemplary embodiments of the disclosure achieves sharply imaged objects 122. In this manner a high xy-resolution of, for example, 50 μm can be achieved.

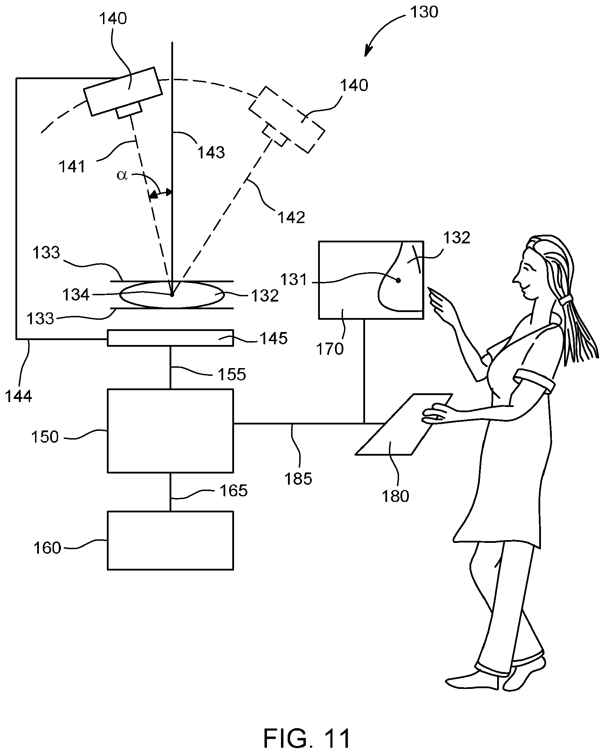

FIG. 11 shows a system for obtaining images of an object of interest, wherein the system 130 comprises an x-ray beam source 140 facing the detector 145. The x-ray beam source 140 and the detector 145 are connected by an arm 144. Between the detector 145 and the source 140 an object of interest 132 can be placed. In the illustrated system, the x-ray source 140 moves in an arc above a single detector 145. Alternatively the source 140 may be held stationary, while one or more detectors 145 are moved or both the source 140 and the detector 145 move. Regardless of the acquisition geometry used, multiple different views of the breast tissue can be acquired via the at least one x-ray source 140. Each of the multiple different views generally corresponds to a different position of the x-ray source 140 and the image receiver in relation to the object of interest 132.

The object of interest 132 is for example a breast compressed by the compression paddles 133. The breast 132 comprises a punctual object 131, which is located in the zero orientation 143. The detector 145 and the x-ray source 140 constitute the acquisition unit, which is connected via data acquisition line 155 to a processing unit 150. The processing unit 150 comprises a memory unit 160, which may be connected via an archive line 165.

A user such as a health professional may input control signals via the user interface 180. Such signals are transferred from the user interface to the processing unit 150 via the signal line 185. Moreover, the user may review the obtained image of the object of interest 132 on the display unit 170 for interesting structures such as the punctual object 131. The method and system according to the disclosure enable the user to obtain access to one or more sharp images of overview slabs having a high x,y-resolution and relatively low z-resolution. Based on these high quality images, the radiologist is capable of identifying all the clinical signs relevant for breast screening. If the health professional is used to mammograms there is the possibility of displaying a 2D like image of the entire volume of the object of interest. Further, the user may decide if the images of 3D volume portions and/or a full-volume image are archived or not. Alternatively, saving and storing the images may occur automatically. Optionally, planes can be down sampled in the xy-resolution before being sent to the archive.

The memory unit 150 can be integral or separate from the processing unit 150. The memory unit 160 allows storage of data such as the 3D volume images and optionally 2D images. In particular, full volume images having a reduced z-resolution, but high x,y-resolution, may be stored for comparison with former mammograms. In general the memory unit 160 may comprise a computer-readable medium, for example, a hard disk or a CD-ROM, diskette a ROM/RAM memory, DVD, a digital source such as a network or the Internet or any other suitable means. The processing unit 150 is configured to execute program instructions stored in the memory unit 160, which cause the computer to perform the methods of the disclosure. One technical effect of performing the methods according to the embodiments of the invention is to reduce processing time and storage space. Due to the reduction of data also the data transfer conditions are improved.

This description uses examples to disclose the invention, including the best mode, and also to enable any person skilled in the art to practice the invention, including making and using any devices or systems and performing any incorporated methods. The patentable scope of the invention is defined by the claims, and may include other examples that occur to those skilled in the art.

LIST OF ELEMENTS

| 100 | stack of tomosynthesis planes |
|-----|-------------------------------|
| 101 | microcalcification |
| 102 | breast |
| 110 | plurality of volume portions |
| 120 | 2D image |
| 121 | image processed according to a non-preferred embodiment |
| 122 | calcifications |
| 125 | image processed according to an embodiment of the disclosure |
| 130 | medical system for obtaining images of an object of interest |
| 131 | structure of interest |
| 132 | object of interest |
| 133 | compression paddles |
| 140 | x-ray source |

| 141 | determined orientation of x-ray beam |
| 143 | zero-orientation |
| 144 | arm |
| 145 | detector |
| 150 | processing unit |
| 155 | data acquisition line |
| 160 | memory unit |
| 165 | archive line |
| 170 | display unit |
| 180 | user interface |
| 185 | signal line |
| 200 | plurality of slabs |
| 202 | breast |
| 210 | plurality of slabs with lower z-resolution than slabs 200 |
| 222 | lesion |
| 220 | 2D-like image |
| 300 | slab image obtained by a non-preferred method |
| 310 | slab obtained by method according to embodiment of invention |
| 330 | plane obtained by method according to embodiment of invention |
| 400 | method according to an exemplary embodiment |
| 401 | method according to another exemplary embodiment |
| 410 | acquiring a plurality of 2D projection images |
| 420 | generation of a 3D volume portion(s) |
| 425 | generating overlapping volume portions |
| 430 | displaying the obtained images |
| 440 | archiving |
| 500 | generating a stack of tomosynthesis planes |
| 510 | volume portion |
| 501 | tomosynthesis plane |
| 520 | displaying selected tomosynthesis plane |
| 521 | microcalcifications on thin tomosynthesis plane |
| 522 | cluster |
| 530 | generating slabbed 3D volume portions |
| 531 | slabbed volume portion |
| 600 | method according to another exemplary embodiment of the disclosure |
| 610 | generating tomosynthesis planes |
| 611 | first tomosynthesis plane |
| 612 | second tomosynthesis plane |
| 613 | third tomosynthesis plane |
| 614 | forth tomosynthesis plane |
| 615 | fifth tomosynthesis plane |
| 620 | high-pass filtering each tomosynthesis plane |
| 630 | projecting on central plane |
| 640 | low-pass filtering the central plane |
| 650 | combining tomosynthesis planes using MIP |
| 660 | slab recombination |
| 670 | displaying final 3D volume portion |
| 700 | slab generation |
| 701 | punctual object in tomosynthesis plane |
| 710 | thin rays |
| 711 | image of punctual object in slabbed 3D volume portion |
| 712 | sharp image of punctual object in slabbed 3D volume portion |
| 730 | slab obtained by a method according to non preferred method |
| 731 | slab after high pass filtering and MIP |
| 732 | slabbed 3D volume after high pass filtering and MIP |
| 750 | slabbed 3D volume generation using MIP and high-pass filter |
| 810 | first central plane |
| 820 | second central plane |

| 830 | third central plane |
| --- | --- |
| 801 | first ray |
| 802 | ray parallel to first ray |
| 811 | sphere |
| 812 | half diagonal d of diamond |
| 813 | radius r |
| 814 | angle $\theta$ |
| 910 | first tomosynthesis plane |
| 911 | object response |
| 920 | central plane |
| 930 | third tomosynthesis plane |
| | |
| T1 | first tomoplane |
| T2 | second tomo plane |
| S1 | first slab |
| S2 | second slab |
| S3 | third slab |
| $Z_S$ | thickness of slab |
| $Z_T$ | thickness of tomo plane |
| x, y | horizontal and vertical axes of 2D plane |
| z | axis perpendicular to 2D plane |
| r | radius |
| $\theta$ | angle |
| $\alpha$ | projection angle |

## Claims

1. A method for obtaining images of an object of interest using a tomosynthesis system comprising an x-ray source facing a detector, the method comprising:

    acquiring (410) a plurality of 2D projection images of the object of interest in a plurality of orientations;
    generating (420) at least one 3D volume portion of the object of interest from the plurality of 2D projection images, wherein the impulse response of a point object is independent of the object's position in the direction perpendicular to the detector (z-axis) of the 3D volume portion; and
    displaying (430) the at least one 3D volume portion;
    wherein at least three temporary tomosynthesis planes are generated comprising a central plane and further tomosynthesis planes composing the at least one 3D volume portion, said generating step further comprising:

        high-pass filtering the tomosynthesis planes composing the 3D volume portion and projecting (630) the said further tomosynthesis planes composing the 3D volume portion on the central plane;
        combining (650) each projected plane with a maximum intensity pixel (MIP) method resulting in MIP processed data;
        low pass filtering (640) the central plane; and
        generating a slabbed image of the 3D volume portion by combining (660) the MIP processed data with the low pass filtered central plane.

2. The method according to claim 1, wherein said generating step (420) comprises:

    reconstructing a plurality of intermediate tomosynthesis planes (610) from the plurality of 2D projection images; and
    using at least some of the intermediate tomosynthesis planes to generate the at least one 3D-volume portion.

3. The method according to any of the preceding claims, wherein the resolution in direction of the z-axis of the 3D volume portion and/or the 3D volume portion thickness is set to an average lesion size.

4. The method according to any of the preceding claims, wherein 3D volume portions are generated with an overlap (425) between two consecutive 3D volume portions.

**5.** The method according to any of the preceding claims, wherein the (x, y) resolution of the 3D volume portion is higher than the (x, y) resolution of the 2D projection images.

**6.** The method according to any of the preceding claims, wherein further a 3D volume portion is generated comprising the entire volume of the object of interest.

**7.** The method according to any of the preceding claims, further comprising: archiving (440) images of the at least one 3D volume portion.

**8.** The method according to any of the precedent claims, wherein said generating step (420) of at least one 3D volume portion comprises a high-pass filtering step (620).

**9.** The method according to any preceding claim,
wherein the cut-off frequency of the high pass filter depends on the (x,y) resolution of the 3D volume portion.

**10.** The method according to any preceding claim,
wherein the cut-off frequency of the high pass filter depends on the 3D volume portion z-resolution and/or thickness.

**11.** The method according to any preceding claim,
wherein the cut-off frequency of the high pass filter depends on the acquisition system tomosynthesis aperture angle.

**12.** A computer program product, which computer program product comprises program instructions for carrying out the steps of the method according to any of claims 1 to 11, when said product is executed on a computer.

**13.** A computer readable medium storing program instructions, which, when executed by a computer cause the computer to perform the method according to any of claims 1 to 11.

**14.** A system for obtaining tomosynthesis images of an object of interest, the system comprising:

an imaging system comprising an x-ray source (140) and a detector (145) configured to acquire a plurality of 2D projection images of the object of interest (132)
a processing unit (150) configured to generate at least one 3D volume portion of the object of interest (132) from the plurality of 2D projection images, wherein the impulse response of a point object (701) is independent of the object's position in the direction perpendicular to the detector (z-axis) of the 3D volume portion; and
a display unit (170) for displaying the at least one 3D volume portion;
wherein at least three temporary tomosynthesis planes are generated comprising a central plane and further tomosynthesis planes composing the at least one 3D volume portion, said generating step further comprising:

high-pass filtering the tomosynthesis planes composing the 3D volume portion and projecting (630) the said further tomosynthesis planes composing the 3D volume portion on the central plane;
combining (650) each projected plane with a maximum intensity pixel (MIP) method resulting in MIP processed data;
low pass filtering (640) the central plane; and
generating a slabbed image of the 3D volume portion by combining (660) the MIP processed data with the low pass filtered central plane.

**Patentansprüche**

**1.** Verfahren zum Erhalten von Bildern eines Gegenstands von Interesse unter Verwendung eines Tomosynthesesystems, das eine Röntgenquelle umfasst, die einem Detektor zugewandt ist, wobei das Verfahren umfasst:

Erfassen (410) mehrerer 2D-Projektionsbilder des Gegenstands von Interesse in mehreren Orientierungen;
Erzeugen (420) mindestens eines 3D-Volumenabschnitts des Gegenstands von Interesse aus den mehreren 2D-Projektionsbildern, wobei die Impulsantwort eines Punktgegenstandes unabhängig von der Position des Gegenstands in der Richtung senkrecht zum Detektor (z-Achse) des 3D-Volumenabschnitts ist; und
Anzeigen (430) des mindestens einen 3D-Volumenabschnitts;
wobei mindestens drei temporäre Tomosynthese-Ebenen erzeugt werden, die eine zentrale Ebene und weitere Tomosynthese-Ebenen umfassen, die den mindestens einen 3D-Volumenabschnitt bilden, wobei der Erzeugungsschritt ferner umfasst:

Hochpassfiltern der Tomosynthese-Ebenen, die den 3D-Volumenabschnitt bilden, und Projizieren (630) der weiteren Tomosynthese-Ebenen, die den 3D-Volumenabschnitt auf der zentralen Ebene bilden;
Kombinieren (650) jeder projizierten Ebene mit einem MIP-Verfahren (MIP = "Maximum Intensity Pixel"), was zu MIP-verarbeiteten Daten führt;
Tiefpassfiltern (640) der zentralen Ebene; und
Erzeugen eines geschichteten Bildes des 3D-Volumenabschnitts durch Kombinieren (660) der MIP-verarbeiteten Daten mit der Tiefpass-gefilterten zentralen Ebene.

2. Verfahren nach Anspruch 1, wobei der Erzeugungsschritt (420) umfasst:
Rekonstruieren mehrerer Tomosynthese-Zwischenebenen (610) aus den mehreren 2D-Projektionsbildern; und Verwenden mindestens einiger der Tomosynthese-Zwischenebenen, um den mindestens einen 3D-Volumenabschnitt zu erzeugen.

3. Verfahren nach einem der vorhergehenden Ansprüche, wobei die Auflösung in Richtung der z-Achse des 3D-Volumenabschnitts und/oder der 3D-Volumenabschnittsdicke auf eine durchschnittliche Läsionsgröße eingestellt ist.

4. Verfahren nach einem der vorhergehenden Ansprüche, wobei 3D-Volumenabschnitte mit einer Überlappung (425) zwischen zwei aufeinanderfolgenden 3D-Volumenabschnitten erzeugt werden.

5. Verfahren nach einem der vorhergehenden Ansprüche, wobei die (x, y) Auflösung des 3D-Volumenabschnitts höher ist als die (x, y) Auflösung der 2D-Projektionsbilder.

6. Verfahren nach einem der vorhergehenden Ansprüche, wobei ferner ein 3D-Volumenabschnitt erzeugt wird, der das gesamte Volumen des Gegenstands von Interesse umfasst.

7. Verfahren nach einem der vorhergehenden Ansprüche, ferner umfassend:
Archivieren (440) von Bildern des mindestens einen 3D-Volumenabschnitts.

8. Verfahren nach einem der vorhergehenden Ansprüche, wobei der Erzeugungsschritt (420) von mindestens einem 3D-Volumenabschnitt einen Hochpassfilterschritt (620) umfasst.

9. Verfahren nach einem der vorhergehenden Ansprüche, wobei die Grenzfrequenz des Hochpassfilters von der (x, y) Auflösung des 3D-Volumenabschnitts abhängt.

10. Verfahren nach einem der vorhergehenden Ansprüche, wobei die Grenzfrequenz des Hochpassfilters von der z-Auflösung und/oder Dicke des 3D-Volumenabschnitts abhängt.

11. Verfahren nach einem der vorhergehenden Ansprüche, wobei die Grenzfrequenz des Hochpassfilters von dem Tomosynthese-Öffnungswinkel des Erfassungssystems abhängt.

12. Computerprogrammprodukt, wobei das Computerprogrammprodukt Programmanweisungen zum Durchführen der Schritte des Verfahrens nach einem der Ansprüche 1 bis 11 umfasst, wenn das Produkt auf einem Computer ausgeführt wird.

13. Computerlesbares Medium, das Programmanweisungen speichert, die, wenn sie von einem Computer ausgeführt werden, den Computer veranlassen, das Verfahren nach einem der Ansprüche 1 bis 11 durchzuführen.

14. System zum Erhalten von Tomosynthesebildern eines Gegenstands von Interesse, wobei das System umfasst:

ein Bildgebungssystem, das eine Röntgenquelle (140) und einen Detektor (145) umfasst, der zum Erfassen mehrerer 2D-Projektionsbilder des Gegenstands von Interesse (132) ausgelegt ist
eine Verarbeitungseinheit (150), die zum Erzeugen mindestens eines 3D-Volumenabschnitts des Gegenstands von Interesse (132) aus den mehreren 2D-Projektionsbildern ausgelegt ist, wobei die Impulsantwort eines Punktgegenstands (701) unabhängig von der Position des Gegenstands in der Richtung senkrecht zu dem Detektor (z-Achse) des 3D-Volumenabschnitts ist; und

13

# EP 3 232 936 B1

eine Anzeigeeinheit (170) zum Anzeigen des mindestens einen 3D-Volumenabschnitts;
wobei mindestens drei temporäre Tomosynthese-Ebenen erzeugt werden, die eine zentrale Ebene und weitere Tomosynthese-Ebenen umfassen, die den mindestens einen 3D-Volumenabschnitt bilden, wobei der Erzeugungsschritt ferner umfasst:

Hochpassfiltern der Tomosynthese-Ebenen, die den 3D-Volumenabschnitt bilden, und Projizieren (630) der weiteren Tomosynthese-Ebenen, die den 3D-Volumenabschnitt auf der zentralen Ebene bilden;
Kombinieren (650) jeder projizierten Ebene mit einem MIP-Verfahren (MIP = "Maximum Intensity Pixel"), was zu MIP-verarbeiteten Daten führt;
Tiefpassfiltern (640) der zentralen Ebene; und
Erzeugen eines geschichteten Bildes des 3D-Volumenabschnitts durch Kombinieren (660) der MIP-verarbeiteten Daten mit der Tiefpass-gefilterten zentralen Ebene.

## Revendications

1. Procédé pour obtenir des images d'un objet d'intérêt au moyen d'un système de tomosynthèse comprenant une source de rayons X faisant face à un détecteur, le procédé comprenant :

l'acquisition (410) d'une pluralité d'images de projection 2D de l'objet d'intérêt dans une pluralité d'orientations ;
la génération (420) d'au moins une partie de volume 3D de l'objet d'intérêt à partir de la pluralité d'images de projection 2D, la réponse d'impulsion d'un objet ponctuel étant indépendante de la position de l'objet dans la direction perpendiculaire au détecteur (axe Z) de la partie de volume 3D ; et
l'affichage (430) de l'au moins une partie de volume 3D ;
au moins trois plans de tomosynthèse temporaires étant générés, comprenant un plan central et des plans de tomosynthèse supplémentaires composant l'au moins une partie de volume 3D, ladite étape de génération comprenant en outre :

le filtrage passe-haut des plans de tomosynthèse composant la partie de volume 3D et la projection (630) desdits plans de tomosynthèse supplémentaires composant la partie de volume 3D sur le plan central ;
la combinaison (650) de chaque plan projeté avec un procédé de pixel d'intensité maximale (MIP) permettant d'obtenir des données traitées de MIP ;
le filtrage passe-bas (640) du plan central ; et
la génération d'une image en tranches de la partie de volume 3D par combinaison (660) des données traitées de MIP avec le plan central filtré passe-bas.

2. Procédé selon la revendication 1, ladite étape de génération (420) comprenant :

la reconstruction d'une pluralité de plans intermédiaires de tomosynthèse (610) à partir de la pluralité d'images de projection 2D ; et
l'utilisation d'au moins certains des plans intermédiaires de tomosynthèse pour générer l'au moins une partie de volume 3D.

3. Procédé selon l'une quelconque des revendications précédentes, la résolution en direction de l'axe Z de la partie de volume 3D et/ou l'épaisseur de la partie de volume 3D étant réglée(s) à une taille moyenne de lésion.

4. Procédé selon l'une quelconque des revendications précédentes, des parties de volume 3D étant générées avec un chevauchement (425) entre deux parties de volume 3D consécutives.

5. Procédé selon l'une quelconque des revendications précédentes, la résolution (x, y) de la partie de volume 3D étant supérieure à la résolution (x, y) des images de projection 2D.

6. Procédé selon l'une quelconque des revendications précédentes, en outre une partie de volume 3D étant générée, comprenant le volume entier de l'objet d'intérêt.

7. Procédé selon l'une quelconque des revendications précédentes, comprenant en outre :
l'archivage (440) des images de l'au moins une partie de volume 3D.

**8.** Procédé selon l'une quelconque des revendications précédentes, ladite étape de génération (420) d'au moins une partie de volume 3D comprenant une étape de filtrage passe-haut (620).

**9.** Procédé selon l'une quelconque des revendications précédentes, la fréquence de coupure du filtre passe-haut dépendant de la résolution (x, y) de la partie de volume 3D.

**10.** Procédé selon l'une quelconque des revendications précédentes, la fréquence de coupure du filtre passe-haut dépendant de la résolution selon Z et/ou de l'épaisseur de la partie de volume 3D.

**11.** Procédé selon l'une quelconque des revendications précédentes, la fréquence de coupure du filtre passe-haut dépendant de l'angle d'ouverture de tomosynthèse du système d'acquisition.

**12.** Produit de programme informatique, lequel produit de programme informatique comprend des instructions de programme pour effectuer les étapes du procédé selon l'une quelconque des revendications 1 à 11, lorsque ledit produit est exécuté sur un ordinateur.

**13.** Support lisible par ordinateur stockant des instructions de programme qui, lorsqu'elles sont exécutées par un ordinateur, amènent l'ordinateur à réaliser le procédé selon l'une quelconque des revendications 1 à 11.

**14.** Système pour obtenir des images de tomosynthèse d'un objet d'intérêt, le système comprenant :

un système d'imagerie comprenant une source de rayons X (140) et un détecteur (145) configuré pour acquérir une pluralité d'images de projection 2D de l'objet d'intérêt (132),
une unité de traitement (150) configurée pour générer au moins une partie de volume 3D de l'objet d'intérêt (132) à partir de la pluralité d'images de projection 2D, la réponse d'impulsion d'un objet ponctuel (701) étant indépendante de la position de l'objet dans la direction perpendiculaire au détecteur (axe Z) de la partie de volume 3D ; et
une unité d'affichage (170) pour afficher l'au moins une partie de volume 3D ;
au moins trois plans de tomosynthèse temporaires étant générés, comprenant un plan central et des plans supplémentaires de tomosynthèse composant l'au moins une partie de volume 3D, ladite étape de génération comprenant en outre :

le filtrage passe-haut des plans de tomosynthèse composant la partie de volume 3D et la projection (630) desdits plans supplémentaires de tomosynthèse composant la partie de volume 3D sur le plan central ;
la combinaison (650) de chaque plan projeté avec un procédé de pixel d'intensité maximale (MIP) permettant d'obtenir des données traitées de MIP ;
le filtrage passe-bas (640) du plan central ; et
la génération d'une image en tranches de la partie de volume 3D en combinant (660) les données traitées de MIP avec le plan central filtré passe-bas.

FIG. 1

FIG. 2

FIG. 3

EP 3 232 936 B1

FIG. 4

FIG. 5

FIG. 6

EP 3 232 936 B1

FIG. 7

EP 3 232 936 B1

FIG. 9

FIG. 8

FIG. 10

FIG. 11

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8285020 B
- US 20120121064 A